(19) Europäisches Patentamt
European Patent Office
Office européen des brevets

(11) Numéro de publication: **0 676 411 A2**

(12) # DEMANDE DE BREVET EUROPEEN

(21) Numéro de dépôt: **95200770.6**

(22) Date de dépôt: **04.06.92**

(51) Int. Cl.[6]: **C07K 5/097**, A61K 38/06, C07K 5/023, C07K 5/027, C07K 5/087, C07K 5/117

Cette demande a été déposée le 28-03-1995 comme demande divisionnaire de la demande mentionnée sous le code INID 60.

Une requête en rectification concernant une page de description manquante a été présentée conformément à la règle 88 CBE. Il est statué sur cette requête au cours de la procédure engagée devant la division d'examen (Directives relatives à l'examen pratiqué à l'OEB, A-V, 3.).

(30) Priorité: **04.06.91 FR 9106721**

(43) Date de publication de la demande: **11.10.95 Bulletin 95/41**

(60) Numéro de publication de la demande initiale en application de l'article 76 CBE : **0 517 589**

(84) Etats contractants désignés: **AT BE CH DE DK ES FR GB GR IT LI LU NL PT SE**

(71) Demandeur: **ADIR ET COMPAGNIE**
**1 rue Carle Hébert**
**F-92415 Courbevoie Cédex (FR)**

(72) Inventeur: **Fauchere, Jean-Luc**
**13, rue d'Orleans**
**92210 Saint-Cloud (FR)**
Inventeur: **Kucharczyk, Nathalie**
**15, Esplanade Raoul Follerau**
**F-92130 Issy Les Moulineaux (FR)**
Inventeur: **Morris, Angela D.**
**63, avenue du Général Leclerc**
**78220 Viroflay (FR)**
Inventeur: **Paladino, Joseph**
**4, rue des Coudriers**
**F-787000 Conflans Sainte-Honorine (FR)**
Inventeur: **Bonnet, Jaqueline**
**19, rue Charcot**
**F-75013 Paris (FR)**
Inventeur: **Thurieau, Christophe**
**84, avenue Kléber**
**F-75116 Paris (FR)**

(54) **Peptides et pseudopeptides dérivés de tachykinines, leur procédé de préparation et les compositions pharmaceutiques qui les contiennent.**

(57) L'invention concerne les dérivés de formule (I) :

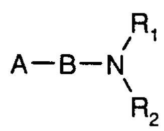

(I)

dans laquelle :

- $R_1$ ou $R_2$ identiques ou différents représentent un atome d'hydrogène, un groupement alkyle, cycloalkyle ou benzyle,
- B représente un résidu d'amino-acide aromatique,
- A représente un résidu peptidique comportant de un à trois amino-acides, sous forme linéaire.

Médicaments.

EP 0 676 411 A2

La présente invention concerne de nouveaux peptides et pseudopeptides dérivés de tachykinines, leur procédé de préparation et les compositions pharmaceutiques qui les contiennent.

Les tachykinines forment une famille de peptides produisant des contractions rapides des fibres musculaires lisses, par opposition aux contractions lentes développées par les bradykinines. La substance P, la neurokinine A et la neurokinine B constituent les principales tachykinines endogènes correspondant respectivement aux récepteurs $NK_1$, $NK_2$ et $NK_3$.

De nombreux peptides antagonistes de tachykinines ont été décrits dans la littérature. C'est le cas par exemple des composés décrits dans les brevets EP-A-333174, EP-A-394989.

La présente invention a pour objet des peptides synthétiques, qui, outre le fait qu'ils soient nouveaux, se sont montrés particulièrement intéressants par l'intensité de leurs propriétés pharmacologiques. Ils possèdent non seulement des propriétés antagonistes puissantes vis-à-vis des récepteurs aux tachykinines et plus particulièrement sélectives et intenses vis-à-vis des récepteurs $NK_1$ c'est-à-dire de substance P. Ces propriétés les rendent utilisables plus particulièrement dans le traitement de la douleur, de l'inflammation, des troubles gastro-intestinaux, de l'asthme, des allergies et des maladies du système nerveux central.

L'invention concerne plus particulièrement de nouveaux dérivés peptidiques répondant à la formule générale (I) :

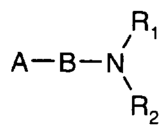

(I)

dans laquelle :

- $R_1$ et $R_2$ identiques ou différents représentent un atome d'hydrogène, un groupement alkyle ($C_1$-$C_6$) linéaire ou ramifié, un groupement cycloalkyle ($C_3$-$C_7$) ou un groupement benzyle,
- B représente un résidu d'amino-acide aromatique,
- A représente un résidu peptidique de formule :

$$P\text{-}A_6\text{-}A_6\text{-}A_4\text{-}$$

dans lequel :

- $A_4$ représente un résidu 2-azabicyclo[2.2.2]octane-3-carbonyl (Abo), leucine (Leu) ou $\beta$-naphtylalanine (Nal),
- $A_6$ représente une liaison ou un résidu phénylalanine (Phe), $\beta$-naphtylalanine (Nal) ou 2-azabicyclo-[2.2.2]octane-3-carbonyl (Abo),
- $A_6$ représente une liaison ou un résidu tryptophane (Trp), tryptophane protégé par un radical formyle (Trp (CHO)), tryptophane protégé par un radical méthyle (Trp ($CH_3$)), ou 2-azabicyclo[2.2.2]-octane-3-carbonyl (Abo),
- P représente un atome d'hydrogène ou un groupement protecteur de la fonction amine tel que benzyloxycarbonyl (Z), tert-butoxycarbonyl (Boc), 3-indolylcarbonyl, benzhydrylcarbonyl ou 9-fluorénylméthyloxycarbonyl (Fmoc),

étant entendu que la liaison peptidique (-CO-NH-) entre $A_6$ et $A_6$, dans le cas où $A_6$ et $A_6$ ne sont pas des liaisons, peut être remplacée par une liaison pseudopeptidique choisie parmi $-CH_2-NH-$ ou $-CH_2-S-$,
leurs énantiomères, diastéréoisomères et épimères ainsi que leurs sels d'addition à un acide ou à une base pharmaceutiquement acceptable,
étant entendu que chaque amino-acide de la séquence peptidique est optiquement pur et que le carbone $\alpha$ de chaque amino-acide peut être de configuration D ou L.

Par résidu d'amino-acide aromatique, on entend plus particulièrement résidu phénylalanine (Phe), tyrosine (Tyr), (1,2,3,4)-tétrahydroisoquinoléine-3-carbonyl (Tic), tryptophane (Trp), tryptophane protégé par un radical formyle (Trp (CHO)) ou pyridinylalanine (Pya).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend aussi au procédé de préparation des composés de formule (I) qui peuvent être obtenus par différentes méthodes telles que la synthèse séquentielle sur phase solide, la synthèse enzymatique, la synthèse génétique par clonage et expression des gènes dans des bactéries transformées ou par des combinaisons diverses de ces techniques.

Les peptides de la présente invention sont généralement obtenus par couplage en solution de fragments peptidiques sélectivement protégés, qui peuvent être préparés soit sur phase solide, soit en solution.

Les méthodes générales de la synthèse des peptides sur phase solide ont été décrites par B.W. ERICKSON et R.B. MERRIFIELD ("The Proteins", Solid-Phase Peptide Synthesis, 3ème édition, 257-527, 1976).

Plus spécifiquement, le procédé de préparation des composés de l'invention suit la méthode de synthèse et de couplage des fragments en solution, bien adaptée à la taille et aux modifications des composés.

Ce procédé est caractérisé en ce que l'on fait réagir un amide de formule (II), optiquement pur :

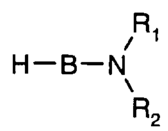

(II)

dans laquelle B, $R_1$ et $R_2$ ont la même signification que dans la formule (I),
avec un deuxième amino-acide, optiquement pur, dont la fonction amine N-terminale est protégée, de formule (III):

$P_1$-$A_4$-OH (III)

dans laquelle :

- $P_1$ représente un groupement protecteur choisi parmi benzyl-oxycarbonyl (Z), tert-butyloxycarbonyl (Boc), 9-fluorényl-méthylcarbonyl (Fmoc),
- $A_4$ a la même signification que dans la formule (I),

en présence d'un réactif de couplage classique de la synthèse peptidique choisi parmi le couple dicyclohexylcarbodiimide (DCC) - hydroxybenzo-triazole (HOBT), le benzotriazol-1-oxytris (diméthylamino) phosphonium hexafluorophosphate (BOP) ou encore le diphénylphosphorylazide (DPPA),
pour conduire, après déprotection sélective de la fonction amine N-terminale, au composé de formule (IV):

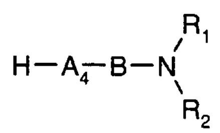

(IV)

dans lesquelles $A_4$, B, $R_1$ et $R_2$ ont les mêmes significations que dans la formule (I),
composé de formule (IV) que l'on fait réagir avec un composé de formule (V) :

$P_1$-$A_6$-$A_6$-OH (V)

dans laquelle $P_1$ a la même signification que précédemment, et $A_6$ et $A_6$ les mêmes significations que dans la formule (I),
(dérivé de formule (V), lui-même obtenu par couplage peptidique classique des amino-acides $A_6$-OH et $A_6$-OH protégés),
en présence d'un réactif de couplage classique de la synthèse peptidique décrit précédemment,
pour conduire, après traitement usuel à un composé de formule (I/a), cas particulier des composés de formule (I):

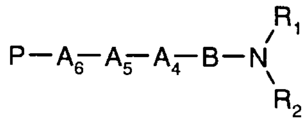

(I/a)

dans laquelle $A_4$, $A_5$, $A_5$, B, $R_1$, $R_2$ et P ont la même signification que dans la formule (I), le radical $A_4$, lorsqu'il représente un résidu tryptophane pouvant être éventuellement protégé par un réactif approprié, contenant le radical Q, par exemple Q-Cl, en présence de réactifs et de solvants convenables dans ce cas, tels que hydroxyde de sodium et sulfate de tétrabutylammonium dans le chlorure de méthylène,

composé de formule (I/a) dont le résidu tryptophane est éventuellement protégé :

que l'on purifie par une technique classique de purification,

que l'on transforme, le cas échéant, en ses sels d'addition à un acide ou à une base pharmaceutiquement acceptable,

qui, lorsqu'il contient une liaison pseudopeptique $-CH_2-NH-$ ou $-CH_2-S-$ est préparé selon le procédé décrit précédemment,

l'introduction de la liaison $-CH_2-NH-$ se faisant en préparant en solution l'aldéhyde $P_1$-NH-CHR-CHO ($P_1$ = groupement protecteur choisi parmi Boc, Fmoc, Z) selon la technique décrite par FEHRENTZ et CASTRO (Synthesis, 676-678, 1983) et en le condensant avec la chaîne peptidique croissante soit sur phase solide, selon la technique décrite par SASAKI et Coy (Peptides, 8, 119-121, 1988), soit en solution, l'introduction de la liaison $-CH_2-S-$ se faisant en condensant le thiol de formule $P_1$-NH-CHR-$CH_2$SH, préparé à partir de l'alcool correspondant, sur le bromure de désamino-peptide C-terminal.

Les composés de l'invention possèdent des propriétés pharmacologiques très intéressantes. Ce sont des ligands spécifiques aux récepteurs des tachykinines qui possèdent des propriétés antagonistes vis-à-vis des récepteurs $NK_1$, $NK_2$ et $NK_3$. Ces propriétés antagonistes sont particulièrement intenses vis-à-vis des récepteurs $NK_1$. Les récepteurs $NK_1$ et $NK_3$ sont impliqués dans la régulation de la transmission de la douleur et l'augmentation de la perméabilité vasculaire. De plus, la stimulation des récepteurs $NK_1$ induit une hypersalivation, celle des récepteurs $NK_2$ entraîne tachycardie, hypotension et bronchoconstriction, enfin celle des récepteurs $NK_3$ est suivie de bradycardie, d'hypotension et de vasodilatation périphérique (D. REGOLI et coll., TIPS, 9, 290-295, 1988).

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols.

La posologie varie selon l'âge et le poids du patient, la nature et la sévérité de l'affection ainsi que la voie d'administration.

Celle-ci peut être orale, nasale, rectale ou parentérale. D'une manière générale, elle s'échelonne entre 0,2 et 100 mg pour un traitement en une ou plusieurs prises par 24 heures.

Les exemples suivants illustrent l'invention et ne la limitent en aucune façon.

Les acides aminés dont les abréviations commencent par une lettre majuscule sont de configuration L.

Les acides aminés dont les abréviations commencent par une lettre minuscule sont de configuration D.

La lettre Ψ indique la présence d'une liaison pseudopeptidique dont la nature est indiquée entre parenthèses.

Les préparations suivantes ne permettent pas d'obtenir les composés de l'invention mais conduisent à des intermédiaires utiles dans la préparation des composés de l'invention.

**PREPARATION A** :

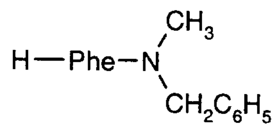

,

$CF_3CO_2H$

60 mmoles de Boc-Phe-OH et 60 mmoles de méthylbenzylamine sont dissoutes dans 150 ml de diméthylformamide. 60 mmoles d'hydroxy-benzotriazole (HOBT) dans 100 ml de diméthylformamide sont ajoutées au mélange précédent puis 80 mmoles de dicyclohexylcarbodiimide (DCC) en solution dans 40 ml de diméthylformamide. L'agitation est maintenue 16 heures à température ambiante.

Après filtration de la dicyclohexylurée formée et évaporation du solvant, le résidu est repris par de l'acétate d'éthyle et la phase organique est lavée par une solution à 5 % d'hydrogénocarbonate de sodium puis par une solution de sulfate de potassium et enfin par une solution saturée de chlorure de sodium.

Après évaporation du solvant, le résidu brut est purifié par chromatographie sur colonne de silice. La fonction amine terminale de la phénylalanine est déprotégée par traitement du composé obtenu pendant 20 minutes dans de l'acide trifluoroacétique, ou par l'acide chlorhydrique gazeux dans l'acétate d'éthyle. Le produit attendu est alors obtenu sous forme de sel.

Rendement : 92 %

**PREPARATION B :**

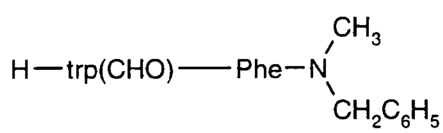

,

$CF_3CO_2H$

50 mmoles du composé obtenu dans la préparation A sont dissoutes dans 100 ml de diméthylformamide en présence de 50 mmoles de triéthylamine. Une solution contenant 50 mmoles de Boc-trp (CHO)-OH, 50 mmoles de HOBT dans 70 ml de diméthylformamide est ajoutée au mélange précédent, suivie par l'addition d'une solution contenant 70 mmoles de DCC dans 30 ml de diméthylformamide. L'ensemble est maintenu 16 heures sous agitation à température ambiante. Le produit attendu sous forme de trifluoroacétate est isolé, purifié et déprotégé procédant comme dans la préparation A.

Rendement : 78 %

**EXEMPLE 1 :**

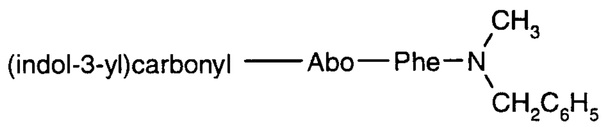

STADE A :

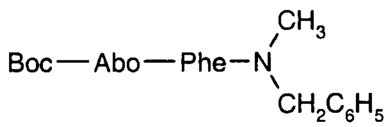

Les exemples suivants ont été obtenus en utilisant le même procédé de synthèse que celui décrit pour l'exemple 1 :

**EXEMPLE 2 :**

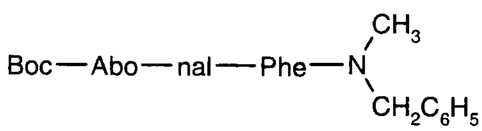

Spectre de masse (FAB) : $MH^+$ : $m/_z$ = 703 (masse moléculaire : 702,9)

**EXEMPLE 3 :**

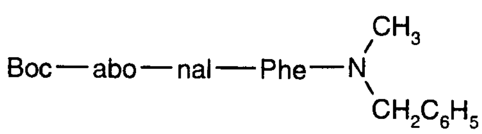

Spectre de masse (FAB) : $MH^+$ : $m/_z$ = 703 (masse moléculaire : 702,9)

**EXEMPLE 4 :** H-trp-Ψ($CH_2S$)-Phe-Abo-Phe-$NH_2$

Les trois exemples suivants ont été obtenus en utilisant le procédé de synthèse sur phase solide, selon Erickson et Merrifield.

**EXEMPLE 5 :** H-trp-Phe-Abo-Phe-$NH_2$, $CF_3CO_2H$

Spectre de masse (FAB) : $MH^+$ : m/z = 635 (masse moléculaire, base libre : 634,8)

**EXEMPLE 6 :** Z-Phe-abo-trp-$NH_2$

Spectre de masse (FAB) : $MH^+$ : m/z = 622 (masse moléculaire : 621,7)

**EXEMPLE 7 :** Benzhydrylcarbonyl-Abo-Leu-Trp-$NH_2$

Spectre de masse (FAB) : $MH^+$ : m/z = 648 (masse moléculaire : 647,8)

## ETUDE PHARMACOLOGIOUE DES DERIVES DE L'INVENTION

**EXEMPLE 8 : Bio-essais sur le muscle lisse isolé**

Afin d'évaluer le potentiel antagoniste des neurokinines des composés de l'invention, trois préparations de muscle lisse ont été utilisées. Chacune de ces préparations, décrite comme présentant une bonne spécificité pour un type de récepteur aux neurokinines, a été réalisée conformément aux techniques suivantes décrites dans la littérature :

- veine cave de lapin pour l'étude du récepteur $NK_1$ selon D.REGOLI et Coll. (J. Cardiovasc. Pharmacol., sous presse);
- artère pulmonaire de lapin sans endothélium pour l'étude de récepteur $NK_2$ selon D.REGOLI et Coll. (European J. Pharmacol. 125, 37-44, 1985) ;
- verte porte de rat pour l'étude du récepteur $NK_3$ selon D.REGOLI et Coll. (European J. Pharmacol. 134, 321-326, 1986).

Le pouvoir antagoniste des composés de l'invention est exprimé sous forme de $pA_2$ tel que défini par O.ARUNLAKSHANA et H.O.SCHILD (Brit. J. Pharmacol., 14, 48-58, 1959).

A titre d'exemple, les résultats suivants ont été obtenus pour l'exemple 1:

| | $pA_2$ | | |
|---|---|---|---|
| **EXEMPLE** | **$NK_1$** | **$NK_2$** | **$NK_2$** |
| 1 | 7,34 | 4,86 | 5,04 |

Les composés de l'invention présentent une puissante activité antagoniste vis-à-vis des récepteurs $NK_1$, avec, pour la plupart, une moindre activité pour les récepteurs $NK_2$ et $NK_3$.

**EXEMPLE 9 : Activité in vivo. Test d'Eddy chez la souris.**

En raison de l'implication de la substance P dans la transmission de la douleur au niveau spinal (M. OTSUKA et S. KONISHI, TINS, 6, 317-320, 1983), l'activité pharmacologique in vivo des composés de l'invention a été recherchée chez la souris sur le test d'hyperalgésie thermique initialement décrit par N.B. EDDY et Coll. (J. Pharmacol. Exp. Ther., 107, 385-393, 1953). Ce test consiste à mesurer le temps de réaction à la chaleur déterminé par le léchage des pattes antérieures chez une souris ($CD_1$ mâle, Ch. River, 25-30 g) placée sur une plaque métallique chauffée à 55°C.

Les animaux ont été traités avec les composés d'invention, 5 minutes avant le passage sur la plaque chauffante par voie intraveineuse.

La moyenne des temps de réaction obtenue pour chaque lot traité (12 souris par lot) a été comparée à la moyenne du lot témoin correspondant. Les résultats sont exprimés sous forme de $DE_{50}$ qui correspond à la dose augmentant de 50 % le temps de réaction.

A titre d'exemple, le composé de l'exemple 1 a fourni les résultats suivants :

| **EXEMPLE** | **Test d'Eddy chez la souris $DE_{50}$ (mg/kg IV)** |
|---|---|
| 1 | 4,0 |
| morphine | 0,5 |

L'activité antalgique des dérivés de l'invention qui mobiliserait tout autre récepteur que ceux connus des neurokinines est également revendiquée.

**COMPOSITION PHARMACEUTIQUE**

**EXEMPLE 10 : Comprimé : formule de préparation pour 1000 comprimés dosés à 2 mg**

| | |
|---|---|
| Composé de l'exemple 1 | 2 g |
| Hydroxypropyl cellulose | 2 g |
| Amidon de blé | 10 g |
| Lactose | 100 g |
| Stéarate de magnésium | 3 g |
| Talc | 3 g |

## Revendications

1. Composés de formule (I):

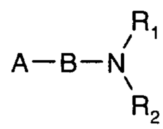

(I)

dans laquelle :

$R_1$ et $R_2$ identiques ou différents représentent un atome d'hydrogène, un groupement alkyle ($C_1$-$C_6$) linéaire ou ramifié, un groupement cycloalkyle ($C_3$-$C_7$) ou un groupement benzyle,

B représente un résidu d'amino-acide aromatique,

A représente un résidu peptidique de formule :

$$P\text{-}A_6\text{-}A_6\text{-}A_4\text{-}$$

dans lequel :

$A_4$ représente un résidu 2-azabicyclo[2.2.2]octane-3-carbonyl (Abo)ou $\beta$ -naphtylalanine (Nal),

$A_6$ représente une liaison, un résidu phénylalanine (Phe), $\beta$-naphtylalanine (Nal) ou 2-azabicyclo-[2.2.2]octane-3-carbonyl (Abo),

$A_6$ représente une liaison, un résidu tryptophane (Trp), tryptophane protégé par un radical formyle (Trp (CHO)), tryptophane protégé par un radical méthyle (Trp ($CH_3$)) ou 2-azabicyclo-[2.2.2]octane-3-carbonyl (Abo),

P représente un atome d'hydrogène ou un groupement protecteur de la fonction amine tel que benzyloxycarbonyl (Z), tert-butoxycarbonyl (Boc), 3-indolylcarbonyl, benzhydrylcarbonyl ou 9-fluorénylméthyloxycarbonyl (Fmoc),

étant entendu que la liaison peptidique (-CO-NH-) entre $A_6$ et $A_6$, dans le cas où $A_6$ et $A_6$ ne sont pas des liaisons, peut être remplacée par une liaison pseudopeptidique choisie parmi -$CH_2$-NH- ou -$CH_2$-S-,
leurs énantiomères, diastéréoisomères et épimères ainsi que leurs sels d'addition à un acide ou à une base pharmaceutiquement acceptable,
étant entendu que chaque amino-acide de la séquence peptidique est optiquement pur et que le carbone $\alpha$ de chaque amino-acide peut être de configuration D ou L.

2. Composés selon la revendication 1 dans lesquels B représente un résidu phénylalanine (Phe), tyrosine (Tyr), (1,2,3,4)-tétrahydroisoquinoléine-3-carbonyl (Tic), tryptophane (Trp) ou tryptophane protégé par un radical formyle (Trp (CHO)), 3-pyridinylalanine (Pya), leurs énantiomères, diastéréoisomères et épimères ainsi que leurs sels d'addition à un acide ou à une base pharmaceutiquement acceptable.

3. Composés selon la revendication 1 dans lesquels A représente un résidu peptidique de formule P-$A_6$-$A_6$-$A_4$- selon la revendication 1, leurs énantiomères, diastéréoisomères et épimères ainsi que leurs sels d'addition à un acide ou à une base pharmaceutiquement acceptable.

**4.** Composé selon la revendication 1 qui est le

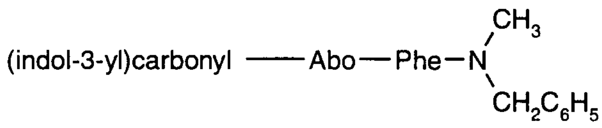

ses énantiomères, diastéréoisomères et épimères.

**5.** Procédé de préparation des composés de formule (I) selon la revendication 1 caractérisé en ce que l'on fait réagir un amide de formule (II), optiquement pur :

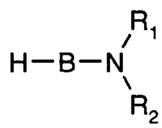

(II)

dans laquelle B, $R_1$ et $R_2$ ont la même signification que dans la formule (I),
avec un deuxième amino-acide, optiquement pur, dont la fonction amine N-terminale est protégée, de formule (III) :

$P_1$-$A_4$-OH (III)

dans laquelle :

$P_1$ représente un groupement protecteur choisi parmi benzyl-oxycarbonyl (Z), tert-butyloxycarbonyl (Boc), 9-fluorényl- méthylcarbonyl (Fmoc),

$A_4$ a la même signification que dans la formule (I),

en présence d'un réactif de couplage classique de la synthèse peptidique choisi parmi le couple dicyclohexylcarbodiimide (DCC)-hydroxybenzo-triazole (HOBT), le benzotriazol-1-oxytris (diméthylamino) phosphonium hexafluorophosphate (BOP) ou encore le diphénylphosphorylazide (DPPA),
pour conduire, après déprotection sélective de la fonction amine N-terminale, au composé de formule (IV):

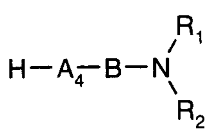

(IV)

dans lesquelles $A_4$, B, $R_1$ et $R_2$ ont les mêmes significations que dans la formule (I),
composé de formule (IV) que l'on fait réagir avec un composé de formule (V):

$P_1$—$A_6$—$A_6$—OH (V)

dans laquelle $P_1$ a la même signification que précédemment, et $A_6$ et $A_6$ les mêmes significations que dans la formule (I),
(dérivé de formule (V), lui-même obtenu par couplage peptidique classique des amino-acides $A_6$-OH et $A_6$-OH protégés),
en présence d'un réactif de couplage classique de la synthèse peptidique décrit précédemment,
pour conduire, après traitement usuel à un composé de formule (I/a), cas particulier des composés de formule (I),

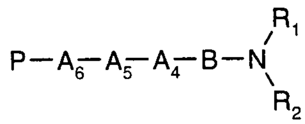

(I/a)

dans laquelle $A_4$, $A_5$, $A_5$, B, $R_1$, $R_2$ et P ont la même signification que dans la formule (I), le radical $A_4$, lorsqu'il représente un résidu tryptophane pouvant être éventuellement protégé par un réactif approprié, contenant le radical Q, défini dans la première revendication,
composé de formule (I/a):
que l'on purifie par une technique classique de purification,
que l'on transforme, le cas échéant, en ses sels d'addition à un acide ou à une base pharmaceutiquement acceptable,
qui, lorsqu'il contient une liaison pseudopeptique $-CH_2-NH-$ ou $-CH_2-S-$ est préparé selon le procédé décrit précédemment,
l'introduction de la liaison $-CN_2-NH-$, se faisant en préparant en solution l'aldéhyde $P_1$-NH-CHR-CHO ($P_1$ = groupement protecteur choisi parmi Boc, Fmoc, Z) et en le condensant avec la chaîne peptidique croissante soit sur phase solide, soit en solution,
l'introduction de la liaison $-CH_2-S-$ se faisant en condensant le thiol de formule $P_1$-NH-CHR-$CH_2$SH, préparé à partir de l'alcool correspondant, sur le bromure de désamino-peptide C-terminal.

**6.** Compositions pharmaceutiques contenant comme principe actif au moins un composé selon l'une quelconque des revendications 1 à 4, seul ou en combinaison avec un ou plusieurs excipients ou véhicules inertes, non toxiques, pharmaceutiquement acceptables.

**7.** Compositions pharmaceutiques selon la revendication 6 contenant au moins un principe actif selon l'une quelconque des revendications 1 à 4 utiles comme antagonistes de substance P dans le traitement de la douleur, de l'inflammation, des troubles gastro-intestinaux, de l'asthme, des allergies et des maladies du système nerveux central.